# EUROPÄISCHE PATENTSCHRIFT

(11) **EP 2 098 168 B1**
(45) Veröffentlichungstag und Bekanntmachung des Hinweises auf die Patenterteilung: **03.11.2010**
(21) Anmeldenummer: 08152242.7
(22) Anmeldetag: 04.03.2008
(51) Int. Cl.: A61B 6/00

(54) **Kalibrierung eines C-Bogen-Röntgengeräts**
Calibration of a C-arm x-ray device
Calibrage d'un appareil de radiographie à cercle en C

(43) Veröffentlichungstag der Anmeldung: 09.09.2009
(73) Patentinhaber: BrainLAB AG, 85622 Feldkirchen (DE)
(72) Erfinder: Maier, Christian, 80802, München (DE); Uhde, Jörg, 80538, München (DE)
(74) Vertreter: Schwabe - Sandmair - Marx

(56) Entgegenhaltungen:
- WO-A-00/47103
- WO-A-2004/052205
- US-B1- 6 379 041
- US-B2- 6 739 752

## Beschreibung

Die Erfindung betrifft die Kalibrierung eines C-Bogen-Röntgengeräts. Insbesondere betrifft sie ein Verfahren für eine solche Kalibrierung sowie ein Verfahren zur Bestimmung der Relativstellung von Projektionszentrum und Bildverstärker eines C-Bogen-Röntgengeräts mit Hilfe einer Kalibrierung.

Bei der durch Fluoroskopie unterstützten, bildgeführten Chirurgie besteht die Notwendigkeit, jedes C-Bogen-Bild zu registrieren, bevor es für Navigationsaufgaben verwendet werden kann. Hierzu wird herkömmlicherweise eine in einem medizintechnischen Trackingsystem lokalisierbare Vorrichtung (Registrierungskit) an dem Bildverstärker des C-Bogens angebracht. Das Registrierungskit hat ein Muster aus strahlungsundurchlässigen Markern, die beim Erstellen des Bildes auf dem C-Bogen-Bild abgebildet werden. Die undurchlässigen Marker müssen in einer solchen Weise angeordnet sein, dass sie nicht vollständig in einer Ebene liegen, was bedeutet, dass sie in einer dreidimensionalen Verteilung vorgesehen werden müssen. Dann kann die relative Position der abgebildeten Marker verwendet werden, um das Projektionszentrum des erstellten Bildes zu bestimmen. Hierzu muss die getrackte Vorrichtung, also das Registrierungskit, eine räumliche Ausdehnung haben und für jedes erstellte Bild auf dem C-Bogen bleiben, weil das Projektionszentrum nämlich für jede Bilderzeugungsrichtung eine andere Relativstellung gegenüber dem Bildverstärker hat. Dies liegt daran, dass der C-Bogen nur eine endliche Steifigkeit hat und in verschiedenen Stellungen verschiedenen Verformungen unterworfen ist.

Die US 6,851,855 B2, die US 6,932,506 B2, die US 7,251,522 B2 und die WO 00/47 103 beschreiben Kalibrierungsvorgänge für einen C-Bogen. Es ist dabei immer notwendig, für jede Bilderfassungsrichtung bei der Kalibrierung auch immer jeweilige Kalibrierungsbilder zu erstellen. Mit anderen Worten: Bei der Kalibrierung muss in jedem Winkel, für den der C-Bogen kalibriert werden soll, auch ein Kalibrierungsbild erstellt werden, um das jeweilige Projektionszentrum zu ermitteln.

Es ist die Aufgabe der vorliegenden Erfindung, die Kalibrierung eines C-Bogen-Röntgengeräts einfacher und insbesondere auch unaufwändiger bzw. schneller zu gestalten. Diese Aufgabe wird erfindungsgemäß durch ein Verfahren gemäß dem Anspruch 1 gelöst. Die Unteransprüche definieren bevorzugte Ausführungsformen der Erfindung.

Gemäß einem Aspekt der Erfindung betrifft diese ein Verfahren zum Kalibrieren eines C-Bogen-Röntgengeräts mit den folgenden Schritten:
a) das C-Bogen-Röntgengerät wird in eine Ausgangsstellung gebracht, insbesondere eine Anterior-Posterior-Stellung;
b) ein Kalibrierungsbild wird erstellt;
c) aus den Informationen des Kalibrierungsbildes wird die Position des Projektionszentrums (P) in der Strahlungsquelle des C-Bogen-Röntgengeräts (4) bestimmt und mit zugeordneten Werten für den Orbitalwinkel (Φ) und den Polarwinkel (ψ) des Bogens des C-Bogen-Röntgengeräts abgespeichert;
d) der Bogen (5) wird in mehrere Kalibrierungsstellungen in einem Bereich von Orbitalwinkeln (Φ) und Polarwinkeln (ψ) bewegt, und für jede Kalibrierungsstellung wird die Relativposition von Strahlungsquelle und Bildverstärker des C-Bogen-Röntgengeräts direkt gemessen;
e) für jede Kalibrierungsstellung wird die relative Positionsänderung von Strahlenquelle zu Bildverstärker des C-Bogen-Röntgengeräts gemessen,
f) die für jede Kalibrierungsstellung geltende Position des Projektionszentrums (P) wird ermittelt aus
   - der Position des Projektionszentrums, die in Schritt c) bestimmt wurde, sowie aus
   - der relativen Positionsänderung von Strahlungsquelle zu Bildverstärker, und
g) für jede Kalibrierungsstellung, d.h. für den jeweiligen Orbitalwinkel (Φ) und den jeweiligen Polarwinkel (ψ), wird die Position des Projektionszentrums (P) abrufbar gespeichert.

Anders als beim Stand der Technik wird also bei dem erfindungsgemäßen Verfahren nicht jedes Mal, das heißt in jeder Kalibrierungsstellung, ein Kalibrierungsbild "geschossen" (mit aufgesetztem Registrierungskit), sondern dieser Vorgang muss nur einmal durchgeführt werden, um festzustellen, wo das Projektionszentrum relativ zur Strahlungsquelle (Bauteil mit der Röntgenröhre an einem Ende des C-Bogens) oder relativ zu einer Trackingreferenz an der Strahlungsquelle liegt. Die Erfindung basiert auf der Erkenntnis, dass das Projektionszentrum selbst sich in seiner relativen Lage in der Strahlungsquelle beziehungsweise gegenüber einer Referenz an der Strahlungsquelle nicht ändern wird, egal in welcher Stellung der C-Bogen sich befindet. Diese Kenntnis ermöglicht es dann, die Position des Projektionszentrums unmittelbar aus der Position der Strahlungsquelle oder der daran befestigten Referenz zu schließen. Wenn dann noch die Position des Bildverstärkers gezeigt wird, lässt sich die Relativstellung von Strahlungsquelle und Bildverstärker feststellen, ohne dass für jeden Kalibrierungswinkel ein eigenes Bild erzeugt werden muss. Anders ausgedrückt: Die oben aufgeführten Schritte b) und c) liefern absolute Koordinaten für das Projektionszentrum (vorzugsweise im Bildverstärker-Koordinatensystem), und der Schritt d) liefert dann die relativen Änderungen dieser Position, die zu den absoluten Koordinaten hinzuaddiert werden müssen, um die neue absolute Position des Projektionszentrums zu erhalten.

Die Erfindung umfasst also ein Verfahren zum Speichern von Informationen über das Projektionszentrum eines Bildes für jede mögliche Bilderstellungsrichtung im Navigationssystem. Diese Information wird dadurch erhalten, dass eine Kalibrierungsprozedur durchgeführt wird, welche es möglich macht, das Bildprojektionszentrum relativ zum Bildverstärker des C-Bogens für jedwede mögliche Bilderstellungsrichtung zu bestimmen. Die Koordinaten des Bildprojektionszentrums werden für jede Bilderstellungsrichtung auf dem Navigationssystem gespeichert, welches für die Fluoroskopie-Navigation bzw. fluoroskopiebildgeführte Behandlung verwendet wird. Während der intraoperativen Verwendung des C-Bogens wird die momentane Bilderstellungsrichtung bestimmt, und aus den gespeicherten Daten kann dann das jeweilige Bildprojektionszentrum aufgefunden werden.

Mit der vorliegenden Erfindung ist es nicht weiter notwendig, in jeder Kalibrierungsstellung eine Vorrichtung an dem Bildverstärker zu befestigen, welche die Bestimmung des Projektionszentrums jedes aufgenommenen Bildes ermöglicht. Da eine solche Vorrichtung eine dreidimensionale Ausdehnung haben muss, nimmt sie gewöhnlich einen gewissen Raum zwischen der C-Bogen-Strahlenquelle und dem Bildverstärker ein, und macht damit den C-Bogen unhandlicher und schwerer bedienbar. Dies kann mit dem erfindungsgemäßen Verfahren vermieden werden. Außerdem werden erfindungsgemäß die Projektionszentren für jede Bilderfassungsrichtung vorab bestimmt, so dass die intraoperative Rechenzeit verringert wird. Außerdem wird die Kalibrierung sehr strahlungsarm, weil nur ein einziges Fluoroskopiebild erstellt werden muss.

Bei einer Ausführungsform der vorliegenden Erfindung wird mindestens eine der Komponenten des C-Bogen-Röntgengeräts durch ein medizintechnisches Trackingsystem räumlich lokalisiert bzw. getrackt, wobei die Trackingdaten insbesondere mit Hilfe eines medizintechnischen Navigationssystems verarbeitet werden. Das Navigationssystem kann auch die sonstigen Daten verarbeiten und bereitstellen, die bei der gesamten Kalibrierung erzeugt oder eingegeben werden.

Wie schon vorher erwähnt wurde, kann die Position des Projektionszentrums im Schritt c) bezogen auf ein Koordinatensystem bestimmt werden, welches fest gegenüber dem Bildverstärker ist.

Es ist möglich, bei der Erstellung des Kalibrierungsbildes am Bildverstärker des Gerätes ein Registrierungskit anzubringen, welches ein Abbildungsmuster aufweist, aus dessen Abbildung im Kalibrierungsbild das Projektionszentrum ermittelt werden kann, und zwar speziell in einem Koordinatensystem des Registrierungskits. Gemäß einer Ausführungsvariante wird das Registrierungskit von außen durch ein medizintechnisches Trackingsystem getrackt oder in bekannter bzw. vorbestimmter Position am trackbaren Bildverstärker des C-Bogen-Röntgengeräts angebracht, so dass das Projektionszentrum im bildverstärkerfesten Koordinatensystem ermittelt werden kann (intern). Die Strahlungsquelle kann ebenfalls von außen durch ein medizintechnisches Trackingsystem getrackt werden, und dies führt dazu, dass auch das Projektionszentrum im strahlungsquellenfesten Koordinatensystem ermittelt werden kann bzw. eine entsprechende Transformation möglich ist.

Das Registrierungskit kann vor dem Schritt der direkten Messung der Relativposition vom C-Bogen entfernt werden, das heißt vom Bildverstärker.

Erfindungsgemäß ist es möglich, die Relativposition von Strahlungsquelle und Bildverstärker des C-Bogen-Röntgengeräts von außen direkt durch daran angebrachte Trackingreferenzen mit dem Trackingsystem zu messen.

Die jeweiligen Orbitalwinkel und die jeweiligen Polarwinkel können in unterschiedlicher Weise gemessen werden, beispielsweise durch
i)
   - direkte Positionsbestimmung für den Bogen mit Hilfe der Trackingreferenzen von Strahlungsquelle und/oder Bildverstärker, und
   - direkte Positionsbestimmung für den C-Bogen-Halter mittels einer an ihm angebrachten Trackingreferenz;
   oder durch
ii)
   - direkte Positionsbestimmung für den C-Bogen-Halter mittels einer an ihm angebrachten Trackingreferenz; und
   - elektr(on)ische, elektromechanische oder mechanische Längsbewegungs- und/oder Winkelsensoren bzw. -abgreifer für die Winkel.

In einer Ausführungsform der Erfindung wird die Positionsänderung der Strahlenquelle gegenüber der Strahlenquellenposition in der Ausgangsstellung ebenfalls wieder durch das Trackingsystem gemessen.

Die Erfindung betrifft gemäß einem anderen Aspekt ein Verfahren zur Bestimmung der Relativstellung von Projektionszentrum und Bildverstärker eines C-Bogen-Röntgengeräts, bei dem die Relativstellung bestimmt wird aus einer für entsprechende Orbitalwinkel und Polarwinkel bestimmten Kalibrierungsstellung, und zwar nach einer Kalibrierung gemäß einem der Verfahren, wie sie oben in unterschiedlichen Ausführungsformen erläutert worden sind.

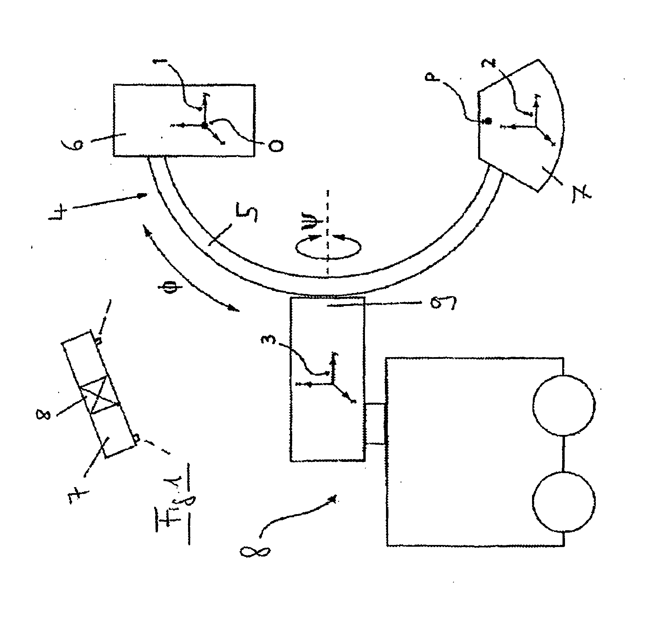

Die Erfindung wird nun anhand eines Ausführungsbeispiels und mit Hilfe der beiliegenden Zeichnungen näher erläutert. Sie kann alle hierin beschriebenen Merkmale einzeln sowie in jeder sinnvollen Kombination umfassen. Die einzige beiliegende Figur 1 zeigt schematisch ein C-Bogen-Röntgengerät im Trackingumfeld.

Das in Figur 1 gezeigte C-Bogen-Röntgengerät 4 weist einen Wagen 8 (C-Bogen-Halter) auf, der ein wagenfestes Koordinatensystem 3 hat. Am Wagen 8 ist der Bogen 5 des C-Bogen-Röntgengeräts 4 befestigt, und zwar so, dass er sich in der Polarrichtung ψ drehen und entlang des Bogens in der Winkelrichtung ϕ verschoben werden kann (Orbitalrichtung). Auf der Unterseite ist am Bogen 5 die Strahlungsquelle 7 mit dem strahlungsquellenfesten Koordinatensystem 2 angebracht. Als Strahlungsquelle 7 gilt hier das Bauteil selbst, das eine ϕRöntgenröhre umfassen wird (nicht im Einzelnen gezeigt). Das Projektionszentrum ist mit dem Buchstaben P markiert und es liegt irgendwo in oder an der Strahlungsquelle. Das Projektionszentrum ist abhängig vom Aufbau der Strahlungsquelle und ihrer Elemente, und es nur schwierig positionsmäßig aus diesem Aufbau zu bestimmen. Besser ist die schon oben beschriebene Bestimmung des Projektionszentrums mittels eines Registrierungskits.

Am oberen Ende des Bogens 5 befindet sich der Bildverstärker 6 mit dem bildverstärkerfesten Koordinatensystem 1 (Mittelpunkt O).

Man kann sich die Koordinatensysteme 1, 2 und 3 auch als Trackingreferenzen vorstellen, beispielsweise als Referenzsteme mit aktiven oder passiven Markern für die Trackingeinheit bzw. das Trackingsystem 7, das nur schematisch dargestellt ist und mit dem das Navigationssystem 8 verbunden ist. Trackingsystem und Navigationssytem 7, 8 sind medizintechnische Systeme, wie sie in der bildgeführten Chirurgie herkömmlicherweise verwendet werden.

Kurz beschrieben basiert die Kalibrierung des C-Bogens 4 auf den folgenden Grundlagen: Zuerst muss die Position des Bildprojektionszentrums P (Koordinatensystem 2) bezüglich der Position des Bildverstärkers (Koordinatensystem 1) bekannt sein. Zweitens muss für eine bestimmte Bilderzeugungsrichtung die Position des Bildverstärkers (Koordinatensystem 1) bezüglich des Wagens 8 (Koordinatensystem 3) bekannt sein. Diese Information über die Bilderzeugungsrichtung und das jeweilige Bildprojektionszentrum kann auf dem Navigationssystem gespeichert werden und während der intraoperativen Verwendung des C-Bogens 4 automatisch abgerufen werden. Dies bedeutet, dass die Bilderzeugungsrichtung zuerst bestimmt werden muss und dann aus den gespeicherten Kalibrierungsdaten abgerufen werden kann, welches Bildprojektionszentrum dieser Bilderzeugungsrichtung zugeordnet ist.

Ganz allgemein gesprochen benötigt die erfindungsgemäße C-Bogen-Kalibrierung die folgenden vier Komponenten:
Erstens eine Vorrichtung, die an der C-Bogen-Strahlungsquelle angebracht ist und es gestattet, ein Koordinatensystem an der Strahlungsquelle aufzuziehen (Koordinatensystem 2). In diesem Koordinatensystem hat das Bildprojektionszentrum P feste aber anfänglich unbekannte Koordinaten. Ein Beispiel für eine solche Vorrichtung wäre eine Trackingreferenz (optisch, elektromagnetisch, ...), die an der Strahlungsquellenabdeckung angebracht ist.

Ein zweites Element ist eine Einrichtung zum Bestimmen der Koordinaten des Bildprojektionszentrums P im Koordinatensystem 2, und hierzu kann beispielsweise ein herkömmliches, getracktes Registrierungskit am Bildverstärker angebracht werden.

Ein drittes Element ist eine Einrichtung zum Bestimmen der Position des Bildverstärkers (Koordinatensystem 1) gegenüber dem C-Bogen-Halter oder Wagen 8 (Koordinatensystem 3), wodurch die Werte der Orbitalwinkel ϕ und der Polarwinkel ψ ermittelt werden. Hierzu können wiederum Trackingmarker am Bildverstärker und am C-Bogen-Halter (Wagen) 8 angebracht werden, oder es werden elektrische, elektromechanische oder mechanische Sensoren genutzt, welche die Orbital- und Polarbewegungen gegenüber der Führung 9 am Wagen 8 messen oder auslesen.

Schließlich wird noch eine Einrichtung benötigt, um die Koordinaten des Koordinatensystems 2 in Koordinaten des Koordinatensystems 1 zu transformieren, und hierzu besteht wiederum die Möglichkeit, Trackingreferenzen an die Strahlungsquelle und den Bildverstärker anzubringen, wobei diese nur während der Kalibrierung benötigt werden. Die rechnerischen Aufgaben für die Transformationen können im Navigationssystem durchgeführt werden.

Mit diesen Vorraussetzungen kann der Ablauf einer erfindungsgemäßen Kalibrierung dann gemäß einem Ausführungsbeispiel wie folgt beschrieben werden:
Der C-Bogen 4 wird zuerst in eine bestimmte, spezifizierte Bilderzeugungsrichtung gebracht, beispielsweise eine AP-Stellung (Anterior-Posterior-Stellung = vertikale Ausrichtung des Bogens 5). Danach wird ein Bild mit einem herkömmlichen Registrierungskit (nicht gezeigt) erstellt, das an dem Bildverstärker angebracht ist. Das Navigationssystem 8 errechnet die Koordinaten des Projektionszentrums P bezüglich der internen Koordinaten des Registrierungskits. Diese Koordinaten werden dann in Koordinaten des Koordinatensystems des Bildverstärkers (Koordinatensystem 1) und in Koordinaten des Koordinatensystems der Strahlungsquelle 7 (Koordinatensystem 2) transformiert. Somit sind die Koordinaten des Bildprojektionszentrums für diese spezielle Bilderzeugungsrichtung (AP) im Koordinatensystem 1 und im Koordinatensystem 2 bekannt. Schließlich werden diese Koordinaten für ϕ (Orbitalwinkel) und ψ (Polarwinkel) für die spezielle Bilderzeugungsrichtung im Navigationssystem gespeichert.

Hierauf wird das Registrierungskit entfernt, während die Trackingreferenzen am Bildverstärker 6, an der Strahlungsquelle 7 und am Wagen 8 noch an ihrem Platz verbleiben.

Der Bogen 5 wird nunmehr durch den möglichen Bewegungsbereich für die orbitale und polare Bewegung hindurch bewegt, und das Trackingsystem 7 zeigt die Positionen der drei Trackingreferenzen (welche die Koordinatensysteme 1, 2 und 3 aufbauen) während dieser gesamten Kalibrierungsprozedur, das heißt die Werte für ϕ und ψ werden zusammen mit der Relativposition von Strahlungsquelle und Bildverstärker jeweils aufgezeichnet. Die Positionskoordinaten der Strahlungsquelle gegenüber dem Bildverstärker werden von den Werten abweichen, die erhalten wurden, als das anfängliche Bild erzeugt wurde, und diese Abweichungen können zu den Koordinaten addiert werden, die anfänglich für das Bildprojektionszentrum erhalten wurden (als das Bild erzeugt wurde). Damit erhält man neue Koordinaten für das Bildprojektionszentrum. Es ist wichtig, dass der gesamte C-Bogen sich für jede aufgezeichnete Position im mechanischen Gleichgewicht des Bogens 5 befindet, so dass restliche Schwingungen der entfernteren Teile die Genauigkeit der Kalibrierung nicht beeinträchtigen.

Wenn die beschriebenen Kalibrierungsschritte durchgeführt worden sind, können die Vorrichtungen zur Bestimmung der Relativposition von Strahlungsquelle und Bildverstärker, also beispielsweise die Trackingreferenzen, abgenommen werden.

Während der intraoperativen Benutzung bestimmt dann das Navigationssystem automatisch die Relativposition von Bildverstärker und C-Bogen-Wagen (Werte für ϕ und ψ), und ruft die zugehörige Position des Bildprojektionszentrums relativ zum Bildverstärker ab, die durch den Kalibrierungsprozess bestimmt wurde.

## Patentansprüche

1. Verfahren zum Kalibrieren eines C-Bogen-Röntgengeräts (4) mit den folgenden Schritten:
a) das C-Bogen-Röntgengerät (4) wird in eine Ausgangsstellung gebracht, insbesondere eine Anterior-Posterior-Stellung;
b) ein Kalibrierungsbild wird erstellt;
c) aus den Informationen des Kalibrierungsbildes wird die Position des Projektionszentrums (P) in der Strahlungsquelle (7) des C-Bogen-Röntgengeräts (4) bestimmt und mit zugeordneten Werten für den Orbitalwinkel (ϕ) und den Polarwinkel (ψ) des Bogens (5) des C-Bogen-Röntgengeräts (4) abgespeichert;
d) der Bogen (5) wird in mehrere Kalibrierungsstellungen in einem Bereich von Orbitalwinkeln (ϕ) und Polarwinkeln (ψ) bewegt, und für jede Kalibrierungsstellung wird die Relativposition von Strahlungsquelle (7) und Bildverstärker (6) des C-Bogen-Röntgengeräts direkt gemessen;
e) für jede Kalibrierungsstellung wird die relative Positionsänderung von Strahlenquelle (7) zu Bildverstärker (6) des C-Bogen-Röntgengeräts gemessen,
f) die für jede Kalibrierungsstellung geltende Position des Projektionszentrums (P) wird ermittelt aus
- der Position des Projektionszentrums (P), die im Schritt c) bestimmt wurde, sowie aus
- der relativen Positionsänderung von Strahlungsquelle (7) zu Bildverstärker (6), und
g) für jede Kalibrierungsstellung, d.h. für den jeweiligen Orbitalwinkel (ϕ) und den jeweiligen Polarwinkel (ψ), wird die Position des Projektionszentrums (P) abrufbar gespeichert.

2. Verfahren nach Anspruch 1, bei dem mindestens eine der Komponenten des C-Bogen-Röntgengeräts (4) durch ein medizintechnisches Trackingsystem (7) räumlich lokalisiert bzw. getrackt wird, wobei die Trackingdaten insbesondere mit Hilfe eines medizintechnischen Navigationssystems (8) verarbeitet werden.

3. Verfahren nach Anspruch 1 oder 2, bei dem die Position des Projektionszentrums (P) im Schritt c) bezogen auf ein Koordinatensystem bestimmt wird, welches fest gegenüber dem Bilderverstärker (6) ist.

4. Verfahren nach einem der Ansprüche 1 bis 3, bei dem im Schritt b) bei der Erstellung des Kalibrierungsbild am Bildverstärker (6) des Geräts (4) ein Registrierungskit angebracht ist, welches ein Abbildungsmuster aufweist, aus dessen Abbildung im Kalibrierungsbild das Projektionszentrum (P) ermittelt werden kann, speziell in einem Koordinatensystem des Registrierungskits.

5. Verfahren nach Anspruch 4, bei dem das Registrierungskit von außen durch ein medizintechnisches Trackingsystem (7) getrackt werden kann oder in bekannter bzw. vorbestimmter Position am trackbaren Bildverstärker (6) des C-Bogen-Röntgengeräts angebracht ist, so dass das Projektionszentrum (P) im bildverstärkerfesten Koordinatensystem (1) ermittelt werden kann.

6. Verfahren nach einem der Ansprüche 1 bis 5, bei dem die Strahlungsquelle von außen durch ein medizintechnisches Trackingsystem (7) getrackt wird, so dass das Projektionszentrum (P) im strahlungsquellenfesten Koordinatensystem (2) ermittelt werden kann.

7. Verfahren nach einem der Ansprüche 1 bis 6, bei dem das Registrierungskit vor dem Schritt d) entfernt wird.

8. Verfahren nach einem der Ansprüche 2 bis 7, bei dem die Relativposition von Strahlungsquelle (7) und Bildverstärker (6) des C-Bogen-Röntgengeräts im Schritt d) von außen direkt durch daran angebrachte Trackingreferenzen mit dem Trackingsystem (7) gemessen wird.

9. Verfahren nach einem der Ansprüche 2 bis 8, bei dem die jeweiligen Orbitalwinkel (ϕ) und die jeweiligen Polarwinkel (ψ) gemessen werden durch
i)
- direkte Positionsbestimmung für den Bogen (5) mit Hilfe der Trackingreferenzen von Strahlungsquelle (7) und/oder Bildverstärker (6), und
- direkte Positionsbestimmung für den C-Bogen-Halter (8) mittels einer an ihm angebrachten Trackingreferenz;
oder durch
ii)
- direkte Positionsbestimmung für den C-Bogen-Halter (8) mittels einer an ihm angebrachten Trackingreferenz; und
- elektr(on)ische, elektromechanische oder mechanische Längsbewegungs- und/oder Winkelsensoren bzw. -abgreifer für die Winkel.

10. Verfahren nach einem der Ansprüche 2 bis 9, bei dem die Positionsänderung im Schritt e) durch das Trackingsystem (7) gemessen wird.

11. Verfahren zur Bestimmung der Relativstellung von Projektionszentrum und Bildverstärker eines C-Bogen-Röntgengeräts, bei dem die Relativstellung bestimmt wird aus einer für entsprechende Orbitalwinkel (ϕ) und Polarwinkel (ψ) bestimmten Kalibrierungsstellung nach einer Kalibrierung gemäß einem der Ansprüche 1 bis 10.

## Claims

1. A method for calibrating a C-arm x-ray apparatus (4), comprising the following steps:
a) the C-arm x-ray apparatus (4) is moved to an initial position, in particular to an anterior-posterior position;
b) a calibration image is produced;
c) the position of the centre of projection (P) in the radiation source (7) of the C-arm x-ray apparatus (4) is determined from the information of the calibration image and stored together with associated values for the orbital angle (ϕ) and the polar angle (ψ) of the arm (5) of the C-arm x-ray apparatus (4);
d) the arm (5) is moved to a number of calibration positions in a range of orbital angles (ϕ) and polar angles (ψ), and the relative position of the radiation source (7) and the image intensifier (6) of the C-arm x-ray apparatus is directly measured for each calibration position;
e) the change in the position of the radiation source (7) relative to the image intensifier (6) of the C-arm x-ray apparatus is measured for each calibration position;
f) the position of the centre of projection (P) which applies to each calibration position is ascertained from:
- the position of the centre of projection (P), as determined in Step c); and from
- the change in the position of the radiation source (7) relative to the image intensifier (6); and
g) the position of the centre of projection (P) is retrievably stored for each calibration position, i.e. for the respective orbital angle (ϕ) and the respective polar angle (ψ).

2. The method according to claim 1, wherein at least one of the components of the C-arm x-ray apparatus (4) is spatially localised and/or tracked by a medical tracking system (7), wherein the tracking data are in particular processed with the aid of a medical navigation system (8).

3. The method according to claim 1 or 2, wherein the position of the centre of projection (P) is determined in Step c) relative to a co-ordinate system which is fixed relative to the image intensifier (6).

4. The method according to any one of claims 1 to 3, wherein when producing the calibration image in Step b), a registration kit is attached to the image intensifier (6) of the apparatus (4), said registration kit comprising an imaging pattern which is imaged in the calibration image, from which the centre of projection (P) can be ascertained, specifically in a co-ordinate system of the registration kit.

5. The method according to claim 4, wherein the registration kit can be tracked from without by a medical tracking system (7) or is attached to the trackable image intensifier (6) of the C-arm x-ray apparatus in a known and/or predetermined position, such that the centre of projection (P) can be ascertained in the co-ordinate system (1) which is fixed relative to the image intensifier.

6. The method according to any one of claims 1 to 5, wherein the radiation source is tracked from without by a medical tracking system (7), such that the centre of projection (P) can be ascertained in the co-ordinate system (2) which is fixed relative to the radiation source.

7. The method according to any one of claims 1 to 6, wherein the registration kit is removed before Step d).

8. The method according to any one of claims 2 to 7, wherein in Step d), the relative position of the radiation source (7) and the image intensifier (6) of the C-arm x-ray apparatus is directly measured from without by tracking references attached to them, using the tracking system (7).

9. The method according to any one of claims 2 to 8, wherein the respective orbital angles (ϕ) and the respective polar angles (ψ) are measured by:
i)
- directly determining the position of the arm (5) with the aid of the tracking references of the radiation source (7) and/or image intensifier (6); and
- directly determining the position of the C-arm holder (8) by means of a tracking reference attached to it;
or by
ii)
- directly determining the position of the C-arm holder (8) by means of a tracking reference attached to it; and
- using electr(on)ic, electromechanical or mechanical longitudinal movement sensors and/or angular sensors and/or tappers for the angles.

10. The method according to any one of claims 2 to 9, wherein the change in position is measured in Step e) by the tracking system (7).

11. A method for determining the relative position of the centre of projection and image intensifier of a C-arm x-ray apparatus, wherein the relative position is determined from a calibration position which is determined for corresponding orbital angles (ϕ) and polar angles (ψ), after a calibration in accordance with any one of claims 1 to 10.

## Revendications

1. Procédé pour calibrer un appareil de radiographie à arceau en C (4) comportant les étapes suivantes:
a) amener l'appareil de radiographie à arceau en C (4) dans une position initiale, en particulier une position antérieure-postérieure,
b) produire une image de calibrage,
c) déterminer la position du centre de projection (P) dans la source de rayonnement (7) de l'appareil de radiographie à arceau en C (4) à partir des informations de l'image de calibrage, et la mémoriser avec des valeurs associées pour l'angle orbital (ϕ) et l'angle polaire (ψ) de l'arceau (5) de l'appareil de radiographie à arceau en C (4),
d) déplacer l'arceau (5) dans plusieurs positions de calibrage dans une plage d'angles orbitaux (ϕ) et d'angles polaires (ψ), et mesurer directement la position relative de la source de rayonnement (7) et de l'intensificateur d'image (6) de l'appareil de radiographie à arceau en C pour chaque position de calibrage, et
e) mesurer la variation relative de position de la source de rayonnement (7) par rapport à l'intensificateur d'image (6) de l'appareil de radiographie à arceau en C pour chaque position de calibrage,
f) déterminer la position qui s'applique à chaque position de calibrage du centre de projection (P) à partir :
- de la position du centre de projection (P) qui a été déterminée à l'étape c), ainsi que
- de la variation relative de position de la source de rayonnement (7) par rapport à l'intensificateur d'image (6), et
g) mémoriser la position du centre de projection (P) de manière à pouvoir la rappeler, pour chaque position de calibrage, c'est-à-dire pour l'angle orbital respectif (ϕ) et l'angle polaire respectif (ψ).

2. Procédé selon la revendication 1, dans lequel au moins un des composants de l'appareil de radiographie à arceau en C (4) est localisé spatialement ou repéré par un système de repérage médico-technique (7), dans lequel les données de repérage sont traitées en particulier à l'aide d'un système de navigation médico-technique (8).

3. Procédé selon la revendication 1 ou 2, dans lequel la position du centre de projection (P) est déterminée à l'étape c) par rapport à un système de coordonnées qui est fixe par rapport à l'intensificateur d'image (6).

4. Procédé selon l'une quelconque des revendications 1 à 3, dans lequel lors de la production de l'image de calibrage à l'étape b), un kit d'enregistrement est fixé sur l'intensificateur d'image (6) de l'appareil (4), ledit kit comportant un modèle d'imagerie dont la reproduction en image permet de déterminer le centre de projection (P) dans l'image de calibrage, en particulier dans un système de coordonnées du kit d'enregistrement.

5. Procédé selon la revendication 4, dans lequel le kit d'enregistrement peut être repéré de l'extérieur par un système de repérage médico-technique (7), ou est fixé dans une position connue ou prédéterminée sur l'intensificateur d'image repérable (6) de l'appareil de radiographie à arceau en C, de telle sorte que le centre de projection (P) peut être déterminé dans le système de coordonnées (1) fixe par rapport à l'intensificateur d'image.

6. Procédé selon l'une quelconque des revendications 1 à 5, dans lequel la source de rayonnement est repérée de l'extérieur par un système de repérage médico-technique (7), de telle sorte que le centre de projection (P) peut être déterminé dans un système de coordonnées (2) fixe par rapport à la source de rayonnement.

7. Procédé selon l'une quelconque des revendications 1 à 6, dans lequel le kit d'enregistrement est retiré avant l'étape d).

8. Procédé selon l'une quelconque des revendications 2 à 7, dans lequel à l'étape d), la position relative de la source de rayonnement (7) et de l'intensificateur d'image (6) de l'appareil de radiographie à arceau en C est directement mesurée de l'extérieur par des références de repérage fixées sur ceux-ci, à l'aide du système de repérage (7).

9. Procédé selon l'une quelconque des revendications 2 à 8, dans lequel les angles orbitaux respectifs (ϕ) et les angles polaires respectifs (ψ) sont mesurés :
i)
- en déterminant directement la position de l'arceau (5) à l'aide des références de repérage de la source de rayonnement (7) et/ou de l'intensificateur d'image (6), et
- en déterminant directement la position du support d'arceau en C (8) au moyen d'une référence de repérage fixée sur celui-ci,
ou
ii)
- en déterminant directement la position du support d'arceau en C (8) au moyen d'une référence de repérage fixée sur celui-ci, et
- en utilisant des capteurs de mouvement longitudinal et/ou des capteurs angulaires ou des capteurs de déplacement angulaire électr(on)iques, électromécaniques ou mécaniques.

10. Procédé selon l'une quelconque des revendications 2 à 9, dans lequel la variation de position à l'étape e) est mesurée par le système de repérage (7).

11. Procédé pour déterminer la position relative d'un centre de projection et d'un intensificateur d'image d'un appareil de radiographie à arceau en C, dans lequel la position relative est déterminée à partir d'une position de calibrage déterminée pour des angles orbitaux (ϕ) et des angles polaires (ψ) correspondants après un calibrage selon l'une quelconque des revendications 1 à 10.
